## Europäisches Patentamt
## European Patent Office
## Office européen des brevets

(11) Veröffentlichungsnummer:

# 0 033 829
## A1

⑫ **EUROPÄISCHE PATENTANMELDUNG**

㉑ Anmeldenummer: 81100043.9

㉒ Anmeldetag: 07.01.81

(51) Int. Cl.³: **C 07 C 76/02, C 07 C 79/46**

㉚ Priorität: 18.01.80 DE 3001695

㊸ Veröffentlichungstag der Anmeldung: 19.08.81
**Patentblatt 81/33**

�[84] Benannte Vertragsstaaten: **CH DE FR GB LI**

㉛ Anmelder: **HOECHST AKTIENGESELLSCHAFT,
Postfach 80 03 20, D-6230 Frankfurt/Main 80 (DE)**

㉒ Erfinder: **Gengnagel, Kurt, Dr., Rossertstrasse 88,
D-6239 Kriftel (DE)**
Erfinder: **Papenfuhs, Theodor, Dr.,
Heinrich-Bleicher-Strasse 40, D-6000 Frankfurt am
Main 50 (DE)**

㉞ Verfahren zur Herstellung von 1-Nitro-benzol-2-carbonsäurealkylester-5-carbonsäuren.

㉗ Verfahren zur Herstellung einer 1-Nitrobenzol-2-carbonsäurealkylester-5-carbonsäure mit einem Alkylrest in der Estergruppe von 1 bis 4 C-Atomen durch saure Esterhydrolyse eines 1-Nitrobenzol-2,5-dicarbonsäuredialkylesters mit Alkylresten in den Estergruppen von jeweils 1 bis 4 C-Atomen, bei welchen die Hydrolyse mittels wäßriger Salpetersäure mit einem Gehalt von insbesondere bevorzugt 10 bis 40% durchgeführt wird. Dieses Verfahren vermeidet die Bildung von explosiblem Dimethyläther gemäß den bekannten Verfahren, der bei der Hydrolyse der Estergruppe aus dem sich bildenden Methanol entsteht.

EP 0 033 829 A1

Verfahren zur Herstellung von 1-Nitro-benzol-2-carbonsäure-
alkylester-5-carbonsäuren

Die Erfindung betrifft eine Verfahrensverbesserung zur Herstellung von wertvollen Zwischenprodukten für Azoverbindungen.

Aus der tschechischen Patentschrift 138 287 ist ein Verfahren zur Herstellung von 1-Nitrobenzol-2-carbonsäuremethylester-5-carbonsäure bekannt, bei welchem eine der Carbonsäuremethylestergruppen in der 1-Nitrobenzol-2,5-dicarbon-
säuredimethylester-Verbindung als Ausgangsverbindung mit
einem großen Überschuß an 70 %iger Schwefelsäure zur Carbonsäuregruppe hydrolysiert wird; die in diesem sauren
Medium abgeschiedene 1-Nitrobenzol-2-carbonsäuremethylester-5-carbonsäure-Verbindung wird sodann mittels Natriumcarbonat gelöst und mit Salzsäure wieder ausgefällt. Diesem Verfahren haftet bei einer technischen Durchführung
der Nachteil einer sehr hohen Abwasserbelastung an. Außerdem erfordert es eine zusätzliche Reinigungsoperation, und
die Bildung von explosiblem Dimethyläther aus dem bei der
Hydrolyse der Estergruppe entstehenden Methylalkohol ist
nicht auszuschließen.

Es wurde nun gefunden, daß man diese Nachteile vermeiden
kann, wenn man bei der Herstellung von 1-Nitrobenzol-2-
carbonsäurealkylester-5-carbonsäuren die saure Esterhydrolyse von 1-Nitrobenzol-2,5-dicarbonsäure-dialkylestern
mittels wäßriger Salpetersäure vornimmt.

Die Erfindung betrifft somit ein Verfahren zur Herstellung
einer 1-Nitrobenzol-2-carbonsäurealkylester-5-carbonsäure
mit einem Alkylrest in der Estergruppe von 1 bis 4 C-Atomen
durch saure Esterhydrolyse eines 1-Nitrobenzol-2,5-dicarbon-
säuredialkylesters mit Alkylresten in den Estergruppen von
jeweils 1 bis 4 C-Atomen, das dadurch gekennzeichnet ist,

daß man Hydrolyse mittels wäßriger Salpetersäure durchführt. - Bevorzugt ist die Herstellung von 1-Nitrobenzol-2-carbonsäuremethylester-5-carbonsäure; man geht hierbei entsprechend von der 1-Nitrobenzol-2,5-dicarbonsäuredimethylester-Verbindung aus.

Der Gehalt der Salpetersäure im wäßrigen Reaktionsmedium kann in weiten Grenzen variieren; im allgemeinen verwendet man eine 5 bis 60 %ige, bevorzugt 10 bis 40 %ige, insbesondere 20 bis 30 %ige Salpetersäure. Die Hydrolyse kann bei Temperaturen von 50 bis 110°C durchgeführt werden; vorzugsweise arbeitet man bei einer Temperatur zwischen 70 und 95°C. Pro Mol Ausgangsverbindung werden in der Regel 1,2 bis 5 Mol, vorzugsweise 1,5 bis 3 Mol Salpetersäure eingesetzt.

Eine bevorzugte Ausführungsform des erfindungsgemäßen Verfahrens ist dadurch gekennzeichnet, daß man die 1-Nitrobenzol-2,5-dicarbonsäuredialkylester-Verbindung in eine etwa 10 bis 40 %ige, vorzugsweise 20 bis 30 %ige, 80 bis 95°C warme Salpetersäure einträgt und dieses Reaktionsgemisch bei dieser Temperatur etwa 7 bis 10 Stunden rührt. Anschließend wird der Reaktionsansatz mit einer wäßrigen Lösung eines Alkalihydroxids, wie beispielsweise Natronlauge, auf einen pH-Wert von etwa 3 bis 3,5 eingestellt, das Gemisch auf eine Temperatur von 5 bis 10°C abgekühlt und noch einige Zeit, beispielsweise eine Stunde, bei dieser Temperatur nachgerührt; die abgeschiedene 1-Nitrobenzol-2-carbonsäurealkylester-5-carbonsäure-Verbindung wird abgesaugt, mit Wasser nachgewaschen und getrocknet.

Das erfindungsgemäße Verfahren schließt eine hohe Abwasserbelastung aus und umgeht bei der Herstellung von 1-Nitrobenzol-2-carbonsäuremethylester-5-carbonsäure die Bildung des Dimethyläthers aus dem entstehenden Methanol, das durch die Salpetersäure oxidiert wird. Ein weiterer Vorteil des erfindungsgemäßen Verfahrens ist die bessere Ausbeute an der 1-Nitrobenzol-2-carbonsäurealkylester-5-carbonsäure-Verbindung und einer besseren Reinheit, die beispielsweise für deren Weiterverarbeitung zu den entsprechenden Carbonsäurechloriden und deren anschließenden Umsetzung zu den entsprechenden Carbonsäurearylamiden ausreichend ist. Die aus diesen 1-Nitrobenzol-2-carbonsäurealkylester-5-carbonsäurearylamiden durch Reduktion erhältlichen 1-Amino-2-carbonsäurealkylester-5-carbonsäurearylamid-Verbindungen können als Diazokomponenten zur Herstellung von wertvollen Azopigmenten (DE-PS 1 263 202) dienen.

Das nachstehende Beispiel dient zur Erläuterung des erfindungsgemäßen Verfahrens. Die Teile sind Gewichtsteile, die Prozentangaben beziehen sich auf Gewichtsprozente, sofern nicht anders vermerkt.

Beispiel:

In eine Mischung aus 800 Teilen 30 %iger wäßriger Salpetersäure und 2 Teilen eines handelsüblichen Entschäumers werden nach dem Erwärmen auf 90°C 200 Teile 1-Nitrobenzol-2,5-dicarbonsäuredimethylester eingetragen. Der Ansatz wird sodann etwa 8 bis 9 Stunden bei einer Temperatur von etwa 90°C gerührt. Der in Suspension befindliche 1-Nitrobenzol-2,5-dicarbonsäuredimethylester schmilzt, und es beginnt sich nach etwa 4 Stunden die 1-Nitrobenzol-2-carbonsäuremethylester-5-carbonsäure als feste Substanz abzuscheiden.

Nach dieser Reaktion werden langsam etwa 350 Teile einer wäßrigen 33 %igen Natronlauge innerhalb einer Stunde bis zu einem pH-Wert von 3,0 bis 3,5 zugetropft, um die als Nebenprodukt entstandene Nitroterephthalsäure in Lösung zu bringen. Hierbei wird durch Außenkühlung eine Temperatur von maximal 10°C eingehalten; es wird sodann noch etwa eine Stunde bei 5 bis 10°C nachgerührt, die 1-Nitrobenzol-2-carbonsäuremethylester-5-carbonsäure abgesaugt, gut trockengesaugt, mit insgesamt 1000 Teilen Wasser in vier Anteilen gewaschen, gut trockengesaugt und getrocknet.

Man erhält 148 Teile 1-Nitrobenzol-2-carbonsäuremethylester-5-carbonsäure mit einem Reingehalt (bestimmt durch quantitative Dünnschichtchromatographie) von etwa 91 %, was einer Ausbeute von 71,6 % der Theorie mit 134,7 Teilen der reinen Verbindung entspricht.

Patentansprüche:

1. Verfahren zur Herstellung einer 1-Nitrobenzol-2-carbonsäurealkylester-5-carbonsäure mit einem Alkylrest in der
   Estergruppe von 1 bis 4 C-Atomen durch saure Esterhydrolyse eines 1-Nitrobenzol-2,5-dicarbonsäuredialkylesters
   mit Alkylresten in den Estergruppen von jeweils 1 bis 4
   C-Atomen, dadurch gekennzeichnet, daß man die Hydrolyse
   mittels wäßriger Salpetersäure durchführt.

2. Verfahren nach Anspruch 1 oder 2, dadurch gekennzeichnet,
   daß man eine 10 bis 40 %ige wäßrige Säure verwendet.

| | EINSCHLÄGIGE DOKUMENTE | | KLASSIFIKATION DER ANMELDUNG (Int. Cl.³) |
|---|---|---|---|
| **Kategorie** | **Kennzeichnung des Dokuments mit Angabe, soweit erforderlich, der maßgeblichen Teile** | **betrifft Anspruch** | |
| D | Chemical Abstracts  Band 76, Nr. 11 13. März 1972 Columbus, Ohio, USA F. MUZIK et al. "Nitromonomethyl terephthalate" Seite 436, Spalte 1, Abstract Nr. 59246g & CS - A - 138 287 ---- | | C 07 C  76/02 C 07 C  79/46 |
| | | | **RECHERCHIERTE SACHGEBIETE (Int. Cl.³)** |
| | | | C 07 C  76/00 C 07 C  76/02 C 07 C  79/38 C 07 C  79/46 |
| | | | **KATEGORIE DER GENANNTEN DOKUMENTE** X: von besonderer Bedeutung A: technologischer Hintergrund O: nichtschriftliche Offenbarung P: Zwischenliteratur T: der Erfindung zugrunde liegende Theorien oder Grundsätze E: kollidierende Anmeldung D: in der Anmeldung angeführtes Dokument L: aus andern Gründen angeführtes Dokument &: Mitglied der gleichen Patent- familie, übereinstimmendes Dokument |

| X | Der vorliegende Recherchenbericht wurde für alle Patentansprüche erstellt. | |
|---|---|---|
| **Recherchenort** Berlin | **Abschlußdatum der Recherche** 14-05-1981 | **Prüfer** PHILLIPS |

EPA form 1503.1  06.78